# EUROPÄISCHE PATENTSCHRIFT

(11) **EP 0 373 443 B1**
(45) Veröffentlichungstag und Bekanntmachung des Hinweises auf die Patenterteilung: **03.03.1993**
(21) Anmeldenummer: 89122186.3
(22) Anmeldetag: 01.12.1989
(51) Int. Cl.: C07C 45/85, C07C 45/82, C07C 47/02, C07C 45/75

(54) **Verfahren zur Gewinnung von 2-alkylverzweigten Alkanalen aus Alkanalgemischen**
Process for obtaining 2-alkyl-branched alkanals from alkanal mixtures
Procédé d'obtention d'alcanals 2-alkyl ramifiés à partir de mélanges d'alcanals

(30) Priorität: 15.12.1988 DE 3842186
(43) Veröffentlichungstag der Anmeldung: 20.06.1990
(73) Patentinhaber: BASF Aktiengesellschaft, 67063 Ludwigshafen (DE)
(72) Erfinder: Roeper, Michael, Dr., D-6706 Wachenheim (DE); Merger, Franz, Dr., D-6710 Frankenthal (DE); Liebe, Joerg, Dr., D-6710 Frankenthal (DE); Grenacher, Armin Volker, Dr., D-6704 Mutterstadt (DE); Hahl, Edgar, Dr., D-6700 Ludwigshafen (DE)

(56) Entgegenhaltungen:
- EP-A- 0 058 927
- EP-A- 0 322 660
- EP-A- 0 335 221
- DE-C- 870 841
- GB-A- 2 078 748

## Beschreibung

Die Erfindung betrifft ein Verfahren zur Abtrennung von 2-alkylverzweigten Alkanalen aus Gemischen, die die 2-alkylverzweigten Alkanale neben n-Alkanalen sowie 3- oder höher alkylverzweigten Alkanalen enthalten. Das Verfahren eignet sich besonders zur Trennung der siedepunktgleichen Verbindungen 2-Methylbutanal und 3-Methylbutanal.

Bei technischen Synthesen von Alkanalen durch Hydroformylierung von Olefinen fallen diese meist nicht in reiner Form sondern als Gemische an. Aus 1-Alkenen, entstehen so die um ein Kohlenstoffatom reicheren Alkanale, wobei je nach Katalysatorsystem in unterschiedlichen Verhältnissen n-Alkanale, 2-Methylalkanale sowie weitere Alkanale entstehen:

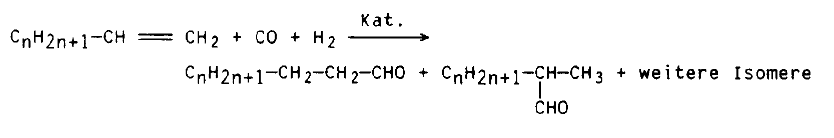

Obwohl meist das n-Alkanal das erwünschte Produkt ist, besteht häufig Interesse daran, auch 2-alkylverzweigte Alkanale zu gewinnen und in hoher Reinheit zu isolieren, da bereits Spuren der isomeren Alkanale bei nachfolgenden Reaktionen empfindlich stören können.

Meist sind jedoch die Siedepunktsunterschiede der isomeren Alkanale so gering, daß eine vollständige Trennung durch Destillation nicht möglich oder unwirtschaftlich ist.

Es bestand daher die Aufgabe, ein Verfahren vorzuschlagen, das es gestattet, isomere Alkanale mit geringen Siedepunktsunterschieden zu trennen.

Eine Zusammenstellung der bisher bekannten Verfahren der Reinigung von Aldehyden kann der Zusammenfassung von J. Weber und P. Lappe in "Methoden der Organischen Chemie (Houben-Weyl)", Georg Thieme Verlag Stuttgart, 1983, Band E 3, S 654, entnommen werden.

Ferner wird in DE 22 18 305 beschrieben, daß langkettige n-Alkanale aus Alkanalgemischen durch Trimerisierung in Gegenwart von Säuren, Abtrennung der festen trimeren n-Alkanale (1,3,5-Trioxane) durch Filtration sowie durch nachfolgende thermische Spaltung über P ₂0₅ rein isoliert werden können.

Für die Gewinnung von 2-Methyldodecanal und von n-Tridecanal wird gemäß DE 28 33 538 eine thermische Behandlung des Isomerengemisches während der Destillation vorgeschlagen. Dabei sollen die geradkettigen Alkanale durch Kondensation in schwerflüchtige Verbindungen übergehen und im Sumpf der Destillation verbleiben, während am Kopf 2-Methyldodecanal abgezogen werden kann. Ob dieses Verfahren auch auf Gemische, welche 3-Alkyl- und höher verzweigte Alkanale enthalten, anwendbar ist, wird nicht ausgeführt.

Jedoch ist bekannt, daß 2-alkylverzweigte Alkanale in Aldolkondensationsreaktionen bedeutend langsamer reagieren als n-Alkanale bzw. 3- oder höher verzweigte Alkanale. Dieser Effekt läßt sich jedoch nicht zur selektiven Abtrennung der 2-alkylverzweigten Alkanale nutzen, da diese in Alkanalgemischen Co-Kondensationsreaktion eingehen (F.J. Doering und G.V. Schaefer, J. Mol. Catal. 41 (1987) 313-328.

Dies wird auch aus den hier vorgelegten Vergleichsbeispielen A und B deutlich, in denen die Gewinnung von 2-Methylbutanal (Kp. 92.5°C/101,3 kPa (760 mm)) aus einem Gemisch mit 3-Methylbutanal (Kp 92-93°C/101,3 kPa (760 mm)) im Verhältnis 92:8 durch basenkatalysierte Kondensation angestrebt wurde. Es zeigte sich jedoch, daß entweder das unerwünschte 3-Methylbutanal nicht vollständig abreagiert (Vergleichsbeispiel B) oder aber der überwiegende Teil des Wertprodukts 2-Methylbutanal durch Kondensation mit 3-Methylbutanal verloren geht (Vergleichsbeispiel A):
Überraschenderweise wurde nun gefunden, daß man 2-alkylverzweigte Alkanale aus Gemischen, die die 2-alkylverzweigte Alkanale neben n-Alkanalen und 3-Alkylalkanalen und weiteren Isomeren enthalten, in hoher Reinheit gewinnen kann, wenn man
a) das Alkanalgemisch mit einer wäßrigen Formaldehydlösung in Gegenwart eines sekundären Amins und einer Carbonsäure als Katalysator bei einem pH-Wert von 2,5 bis 7 umsetzt,
b) die wäßrige Phase mit dem Katalysator und gegebenenfalls nicht umgesetzten überschüssigem Formaldehyd abtrennt und
c) die 2-alkylverzweigten Alkanale durch Destillation von den Kondensationsprodukten der übrigen Alkanale mit Formaldehyd gewinnt

Die neue Reinigungsmethode findet vor allem Anwendung auf das Gemisch aus Valeraldehyd mit seinen Isomeren, aus dem nach Vorabtrennung des Valeraldehyds das 2-Methylbutanal gewonnen wird.

Für die Umsetzung mit Formaldehyd kommen die an sich bekannten Bedingungen einer Mannichkondensation in Betracht. Insbesondere sind die Bedingungen des Verfahrens der DE 3 106 557 geeignet, auf die hiermit Bezug genommen wird. Demgemäß werden vorzugsweise solche Mengen wäßrigen Formaldehyds eingesetzt, daß die mindestens stöchiometrische Menge Formaldehyd (ber. 100 %) bezogen auf die Summe der in α-Stellung unsubstituierten Aldehyde des Gemisches vorliegt und daß man 0,05 bis 1,5 Äquivalente Carbonsäure, d. s. Mono- und Dicarbonsäuren, und 0,05 bis 1,5 Mol Amin, jeweils bezogen auf ein Mol eingesetzten Formaldehyd (ber. 100 %) anwendet und die Umsetzung mit Formaldehyd bei einem pH von 2,5 bis 7 durchführt.

Die in 2-Stellung unsubstituierten Alkanale des Gemisches können jedoch allgemein mit Formaldehyd im Unterschuß oder Überschuß, vorzugsweise in der etwa einmolaren Menge oder im überschuß, d.i. in einer Menge von 0,9 bis 10, vorzugsweise 0,95 bis 5, insbesondere 1,0 bis 2 Mol Formaldehyd je Mol in 2-Stellung unsubstituierte Alkanale umgesetzt werden.

Der Formaldehyd wird zweckmäßig in wäßriger, vorteilhaft in 20 bis 60-gewichtsprozentiger Lösung verwendet. Als Säuren verwendet man aliphatische Mono-, Di- und Polycarbonsäuren mit 2 bis 10 Kohlenstoffatomen. Die Dicarbonsäuren und Polycarbonsäuren (darunter vorzugsweise Tricarbonsäuren) können auch aromatische und araliphatische Carbonsäuren sein. Dicarbonsäuren und Polycarbonsäuren sind vorteilhafter als Monocarbonsäuren. Geeignet sind z.B.: Essigsäure, Propionsäure, Methoxyessigsäure, Buttersäuren, Pentan-, Hexan-, Heptan-, Octan-, Nonan-, Decansäuren, Pivalinsäure, 2-Ethylbuttersäure, 2-Methylpentansäure, 2-Ethylhexansäure, Isononansäure; Pimelinsäure, Korksäure, Acelainsäure, Sebacinsäure, Nonandicarbonsäure, Decandicarbonsäure, Butantetracarbonsäure, Pentan-1,3,5-tricarbonsäure, 3-Hydroxyglutarsäure, Zuckersäure, α,α′-Dihydroxyadipinsäure, bevorzugt Oxalsäure, Bernsteinsäure, Glutarsäure, Adipinsäure, Äpfelsäure, Weinsäure, Butan-1,2,4-tricarbonsäure, 3-Ethylpentan-1,3,5-tricarbonsäure, Zitronensäure, Trimellitsäure, Butantetracarbonsäure, Pyromellitsäure, Phthalsäure, Terephthalsäure, Isophthalsäure, Fumarsäure und besonders bevorzugt Oxalsäure.

Als Amine kommen zweckmäßig solche der Formel 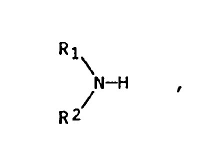
in Betracht, worin R₁ und R² gleich oder verschieden sein können und einen, vorteilhaft mit einem oder mehreren Heteroatomen, bevorzugt mit Hydroxyl und/oder sekundärem bzw. tertiärem Amin substituierten Alkylrest mit 1 bis 12, vorteilhaft 1 bis 10, bevorzugt 1 bis 6 Kohlenstoffatomen bedeuten, R² und R³ mit dem benachbarten Kohlenstoffatom auch Glieder eines vorteilhaft 5- oder 6-gliedrigen Ringes, der noch ein Stickstoff- oder Sauerstoffatom enthalten kann, bilden können. R² kann auch den Rest 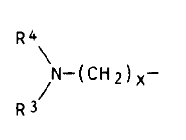
bedeuten, worin R³ und R⁴ gleich oder verschieden sein können und jeweils einen, gegebenenfalls durch mehrere, vorteilhaft zwei, bevorzugt eine Hydroxygruppe substituierten Alkylrest mit 2 bis 18, zweckmäßig 2 bis 10, bevorzugt 2 bis 6 Kohlenstoffatomen bezeichnen, R₃ auch für ein H-Atom stehen kann und x eine Zahl von 2 bis 6 bedeutet. Vorteilhaft steht bei Monohydroxyalkylresten die Hydroxygruppe in ω-Stellung. Bevorzugt werden sekundäre Amine mit einem Siedepunkt oberhalb von 130°C. Besonders bevorzugt werden sekundäre Amine mit sehr geringer Flüchtigkeit wie solche Hydroxyalkylamine, die man z.B. aus Ammoniak oder primären Aminen mit Alkylenoxiden leicht gewinnen kann oder Amine mit zwei oder mehreren Aminogruppen, von denen mindestens eine sekundär, die anderen sekundär und/oder tertiär sind.

So kommen als sekundäre Amine im einzelnen in Betracht: N-Methyl-, N-Ethyl-, N-Propyl-, N-Isopropyl-, N-Butyl-, N-Isobutyl-, N-sek.-Butyl-, N-tert.-Butyl-, N-Pentyl-, N-Hexyl-, N-Heptyl-, N-Octyl-, N-Nonyl-, N-Decyl-(hydroxyethylamin); entsprechende durch einen vorgenannten Substituenten gleich oder unterschiedlich zweimal substituierte Amine; Piperidin, Morpholin, Pyrrolidin, Piperazin, N-Methylpiperazin; N-Ethyl-, N-Propyl-, N-Isopropyl-, N-Butyl-, N-Isobutyl-, N-sek.-Butyl-, N-tert.-Butyl-, N-Pentyl-, N-Hexyl-, N-Heptyl-, N-Octyl-, N-Nonyl-, N-Decyl-N-Hydroxyethyl-amin; entsprechende durch einen vorgenannten hydroxylfreien Substituenten und durch die Hydroxypropyl- und Hydroxybutyl-Gruppe substituierte Amine; gleich oder unterschiedlich durch vorgenannte Hydroxyalkylsubstituenten zweimal substituierte Amine N,N,N′-Triethanolethylendiamin, N,N′-Diethanolethylendiamin und homologe Di- bzw. Tripropanol-Verbindungen bzw. entsprechende Propylen- und Butylendiamin-Verbindungen. Bevorzugt sind Methylhydroxyethylamin, Ethylhydroxyethylamin, Isopropylhydroxyethylamin, Butylhydroxyethylamin, Isobutylhydroxyethylamin, Methylhydroxypropylamin, Ethylhydroxypropylamin, Propylhydroxypropylamin, Isopropylhydroxypropylamin, Butylhydroxypropyl amin, Isobutylhydroxypropylamin, Dihydroxyethylamin, Dihydroxypropylamin, N,N′-Diethanolethylendiamin, Piperazin, N-Methylpiperazin, Dibutylamin.

Man verwendet im Falle von Monocarbonsäuren vorteilhaft zwischen 0,05 bis 1,5, zweckmäßig 0,06 bis 1,4, bevorzugt 0,03 bis 1,25, insbesondere 0,6 bis 1,1 Äquivalente Monocarbonsäure und vorteilhaft 0,05 bis 1,5, bevorzugt 0,3 bis 1,5, insbesondere 0,6 bis 1,2 Äquivalente Amine je Mol Formaldehyd. Man verwendet im Falle von Di- oder Polycarbonsäuren vorteilhaft zwischen 0,01 bis 1,5, bevorzugt 0,05 bis 1,5, insbesondere 0,3 bis 1,25 Äquivalente Di- bzw. Polycarbonsäure und vorteilhaft 0,01 bis 1,5, bevorzugt 0,05 bis 1,5, insbesondere 0,3 bis 1,25 Äquivalente Amine je Mol Formaldehyd. Das Verhältnis Carbonsäure zu Amin beträgt 1 bis 2, vorzugsweise 1,05 bis 1,8, insbesondere 1,1 bis 1,5 Äquivalente Carbonsäure je Äquivalent Amin.

Die Umsetzung wird bei einem pH von 2,5 bis 7, vorzugsweise 3 bis 6,5, insbesondere 3 bis 6, einer Temperatur von 0°C bis 150°C, vorteilhaft 20 bis 130°C, bevorzugt 30 bis 120°C, insbesondere 40 bis 110°C und bei Atmosphärendruck oder unter erhöhtem oder vermindertem Druck kontinuierlich oder diskontinuierlich durchgeführt.

Die Reaktion kann wie folgt durchgeführt werden: Das Alkanalgemisch wird zusammen mit Amin, Formaldehyd, Wasser und Säure während 1 - 300 Minuten, in der Regel 5 - 120 Minuten, vorzugsweise 10 - 90 Minuten bei der Reaktionstemperatur gehalten. Dann wird aus dem Reaktionsgemisch durch Phasentrennung und Destillation der organischen Phase das 2-Alkyl-alkanal gewonnen.

Wie am Beispiel des Methylbutanalgemisches gezeigt, gelingt es unter den erfindungsgemäßen Bedingungen aus einem 92:8 Gemisch von 2- und 3-Methylbutanal selektiv und vollständig das 3-Methylbutanal mit Formaldehyd zum Isopropylacrolein umzusetzen, während das 2-Methylbutanal unverändert bleibt, gemäß der folgenden Reaktion:

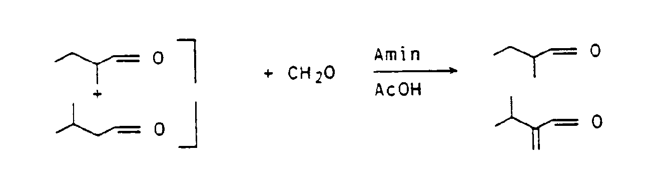

Nach Reaktionsende wird die wäßrige Phase, welche unumgesetzten Formaldehyd sowie den Katalysator (Carbonsäure, sekundäres Amin) enthält, abgetrennt. Sie kann - nach Ergänzung von Formaldehyd - erneut eingesetzt werden.

Aus der organischen Phase kann nunmehr 2-Methylbutanal ohne weitere Schwierigkeiten destillativ in hoher Reinheit, in der Regel über 99 % abgetrennt werden, da der Siedepunktsunterschied zum Isopropylacrolein (Kp 108,5°C/101.3KPa (760 mm)) 16°C beträgt. Das im Sumpf verbleibende Isopropylacrolein kann - gegebenenfalls nach Destillation - ebenfalls genutzt werden.

Das Verfahren läßt sich auf 2-Alkylalkanale mit bis zu 20°C-Atomen, bevorzugt auf C₄ bis C₁₃-Alkanale zur Abtrennung von den Alkanalen gleicher C-Zahl anwenden, welche insbesondere bei langkettigen Alkanalen destillativ schwierig ist. So kann man 2-Methyldecanal (Kp 63-64°C/0,5 mbar) aus einem Gemisch mit n-Undecanal (Kp 64-66°C/0,5 mbar), wie es bei der Hydroformylierung von 1-Decen anfällt gewinnen. Die destillative Abtrennung des 2-Methyldecanals gelingt problemlos, da das aus dem n-Undecanal selektiv gebildete 2-Nonylacrolein mit (Kp 80-82°C/0,5 mbar) einen deutlich höheren Siedepunkt aufweist. Das im Sumpf verbleibende 2-Nonylacrolein kann - gegebenenfalls nach Destillation - ebenfalls genutzt werden.

Die Beispiele zeigen, daß die Abtrennung der 2-alkylverzweigten Alkanale durch das erfindungsgemäße Verfahren unabhängig von der Kettenlänge des Alkanals bzw. des Alkylrestes in 2-Stellung wesentlich erleichert wird bzw. überhaupt erst ermöglicht wird.

### Beispiele

### Beispiel A (Vergleichsbeispiel)

### Versuch der Entfernung von 3-Methylbutanal aus 2-Methylbutanal durch Kondensation mittels methanolischer KOH

Zu 100 g eines Aldehydgemisches, bestehend aus 92,2 % 2-Methylbutanal, 7,3 % 3-Methylbutanal und 0,2 % n-Pentanal wurden in einem Kolben, der mit Magnetrührer, Tropftrichter, Rückflußkühler und Innenthermometer ausgestattet war, 10 ml einer 15 %igen methanolischen Lösung von KOH langsam zugetropft. Die Innentemperatur stieg dabei von Raumtemperatur rasch auf 55°C an und wurde mittels eines Eisbads zwischen 50 und 55°C gehalten. Nachdem die Kalilauge vollständig zugetropft war, wurde noch eine Stunde bei 50°C gerührt, dann mit 200 ml Wasser gewaschen und gaschromatographisch analysiert. Das Produkt enthielt 0,1 % 3-Methylbutanal, 32,4 % 2-Methylbutanal sowie 66,9 % hochsiedende Kondensationsprodukte. Die anschließende Destillation über eine 50 cm-Füllkörperkolonne bei Normaldruck lieferte als Hauptfraktion bei 92,5°C 26,1 g 2-Methylbutanal mit einem Gehalt von 99,5 % sowie 0,4 % an 3-Methylbutanal.

Demnach konnten zwar die unerwünschten Produkte 3-Methylbutanal und n-Pentanal weitgehend aus dem Gemisch entfernt wurden, gleichzeitig gehen jedoch etwa 60 % des Wertproduktes 2-Methylbutanal als Kondensationsprodukt verloren.

### Beispiel B (Vergleichsbeispiel)

### Versuch der Entfernung von 3-Methylbutanal aus 2-Methylbutanal durch Kondensation mittels einer verminderten Menge an methanolischer KOH

Zu 100 g eines Aldehydgemisches gleicher Zusammensetzung wie in Vergleichsbeispiel A wurden in der gleichen Apparatur 2,4 ml einer 15 %igen Lösung von KOH in Methanol zugetropft. Durch Kühlen mit einem Eisbad wurde die Temperatur bei 50 bis 55°C gehalten. Nach vollständiger Zugabe der KOH-Lösung wurde noch eine Stunde bei 50°C gerührt, dann auf Raumtemperatur gekühlt, mit Eisessig neutral gestellt und mit Wasser gewaschen. Die gaschromatographische Analyse ergab einen Gehalt von 1,1 % 3-Methylbutanal, 64,4 % 2-Methylbutanal, 0,01 % n-Pentanal und 33,4 % hochsiedende Kondensationsprodukte.

Obwohl bereits etwa 28 % des Wertproduktes 2-Methylbutanal als hochsiedende Kondensationsprodukte verloren gingen, war die Umsetzung des 3-Methylbutanals nicht vollständig. Auf die Destillation des Produktes wurde daher verzichtet.

Demnach gelingt es mit Hilfe einer basenkatalysierten Aldolkondensation wegen zu geringer Selektivität der Reaktion nicht, 2-alkylverzweigte Alkanale in hoher Ausbeute und damit wirtschaftlich abzutrennen.

### Beispiel 1

### Gewinnung von 2-Methylbutanal aus einem Pentanalgemisch

Zu einem Gemisch aus 15 g Methylethanolamin (0,2 mol), 12 g Essigsäure (0,2 mol) und 20 g 30 %iger Formaldehydlösung (0,2 mol) wurden bei 50°C 100 g eines Aldehydgemisches bestehend aus 91,5 Gew.-%. 2-Methylbutanal, 7,8 Gew.-% 3-Methylbutanal und 0,2 Gew.-% n-Pentanal (3-Methylbutanal -+ n-Pentanal: 0,10 mol) gegeben. Nach einstündigem Rühren bei 50°C wurde eine Probe der organischen Phase gaschromatographisch analysiert; es wurden 89,6 Gew.-% 2-Methylbutanal, 10,5 Gew.-% Isopropylacrolein (Kondensationsprodukt aus 3-Methylbutanal und Formaldehyd) sowie 0,2 Gew.-% eines weiteren Produktes, vermutlich Propylacrolein (Kondensationsprodukt aus n-Pentanal und Formaldehyd) gefunden. Die anschließende Destillation über eine 50 cm-Füllkörperkolonne (Drahtnetzringe) bei Normaldruck liefert als Hauptfraktion bei 92,5°C 83,7 g 2-Methylbutanal mit einem Gehalt von 99,6 Gew.-%.

### Beispiel 2

### Gewinnung von 2-Methyldecanal aus Undecanalgemischen

Zu einem Gemisch aus 112,5 g 40%iger wäßriger Dimethylamin-Lösung (1 mol) 60 g Essigsäure (1 mol) und 110 g 30 gew.%iger Formaldehydlösung (1,1 mol) wurden bei 55 bis 60°C 243 g eines Aldehydgemisches bestehend aus 70%igem n-Undecanal (1 mol) und 30 gew.-%igem 2-Methyldecanal gegeben. Nach 3 Stunden Rühren bei dieser Temperatur zeigte eine gaschromatographische Analyse der organischen Phase, daß das n-Undecanal vollständig zum 2-Nonylacrolein umgesetzt ist, während das 2-Methyldecanal unverändert vorliegt. Die Zusammensetzung betrug: 71,5 Gew.% 2-Nonylacrolein und 28,5 Gew.-% 2-Methyldecanal. Die anschließende Destillation über eine 60 cm-Füllkörperkolonne (Drahtnetzringe) bei 0,5 mbar lieferte als Vorlauf bei 63 bis 64°C 62 g 2-Methyldecanal mit einer Reinheit von 99,3 %. Im Sumpf verblieb 2-Nonylacrolein.

### Beispiel 3

### Gewinnung von 2-Methylhexanal aus Heptanalgemischen

Zu einer Lösung von 75 g Methylethanolamin (1 mol), 60 g Essigsäure (1 mol) in 210 g 30 gew.-%iger Formaldehydlösung (2,1 mol) wurden bei 50 bis 60°C 386 g eines Gemisches aus 41 Gew.-% 2-Methylhexanal und 59 Gew.-% n-Heptanal (Sdp. 42 bis 45°C/10 mbar) unter gutem Rühren gegeben. Nach 4 Stunden bei 60°C wurde die organische Phase gaschromatographisch analysiert. Die organische Phase enthält 38,5 Gew.-% 2-Methylhexanal und 61 Gew.-% 2-Pentylacrolein. Die anschließende Destillation über eine 60 cm - Füllkörperkolonne ergab 139 g 2-Methylhexanal (Sdp. 39 bis 40°C/10 mbar) mit einer Reinheit von 99,2 Gew.-%.

## Patentansprüche

1. Verfahren zur Gewinnung von 2-alkylverzweigten Alkanalen aus Gemischen, die die 2-alkylverzweigten Alkanale neben n-Alkanalen und 3-Alkylalkanalen und weiteren Isomeren enthalten, dadurch gekennzeichnet, daß man
a) das Alkanalgemisch mit einer wäßrigen Formaldehydlösung in Gegenwart eines sekundären Amins und einer Carbonsäure als Katalysator bei einem pH-Wert von 2,5 bis 7 umsetzt,
b) die wäßrige Phase mit dem Katalysator und gegebenenfalls nicht umgesetzten überschüssigen Formaldehyd abtrennt und
c) die 2-alkylverzweigten Alkanale durch Destillation von den Kondensationsprodukten der übrigen Alkanale mit Formaldehyd abtrennt.

2. Verfahren gemäß Anspruch 1, dadurch gekennzeichnet, daß man Formaldehyd in der etwa einmolaren Menge oder im überschuß, bezogen auf die Summe der in 2-Stellung unsubstituierten Alkanale anwendet.

3. Verfahren gemäß Anspruch 2, dadurch gekennzeichnet, daß man 0,05 bis 1,5 Äquivalente Carbonsäure und 0,05 bis 1,5 Mol Amin, bezogen auf ein Mol eingesetzten Formaldehyd (ber. 100 %), anwendet und die Umsetzung mit Formaldehyd bei einem pH vom 2,5 bis 7 durchführt.

4. Verfahren gemäß Anspruch 1, dadurch gekennzeichnet, daß man 2-Methylbutanal aus dem Gemisch der isomeren Methylbutanale mit Valeraldehyd gewinnt.

## Claims

1. A process for obtaining alkanals having a 2-alkyl branch from mixtures which contain alkanals having a 2-alkyl branch in addition to n-alkanals and 3-alkyl-alkanals and further isomers, wherein
a) the alkanal mixture is reacted with an aqueous formaldehyde solution in the presence of a secondary amine and of a carboxylic acid as a catalyst at a pH of from 2.5 to 7,
b) the aqueous phase containing the catalyst and any unconverted excess formaldehyde is separated off and
c) the alkanals having a 2-alkyl branch are isolated by distillation from the condensates of the other alkanals with formaldehyde.

2. A process as claimed in claim 1, wherein formaldehyde is used in about a molar amount or in excess, based on the sum of the 2-unsubstituted alkanals.

3. A process as claimed in claim 2, wherein from 0.05 to 1.5 equivalents of carboxylic acid and from 0.05 to 1.5 moles of amine, based on one mole of formaldehyde used (calculated as 100%), are employed and the reaction with formaldehyde is carried out at a pH of from 2.5 to 7.

4. A process as claimed in claim 1, wherein 2-methylbutanal is obtained from the mixture of the isomeric methylbutanals with valeraldehyde.

## Revendications

1. Procédé d'obtention d'alcanals à ramification 2-alkylique à partir de mélanges qui contiennent les alcanals à ramification 2-alkylique, outre des n-alcanals et des 3-alkylalcanals et d'autres isomères, caractérisé en ce que
a) on fait réagir le mélange d'alcanals avec une solution aqueuse de formaldéhyde, en présence d'une amine secondaire et d'un acide carboxylique servant de catalyseur, à un pH de 2,5 à 7,
b) on sépare la phase aqueuse avec le catalyseur et le formaldéhyde excédentaire éventuellement non entré en réaction et
c) on sépare les alcanals à ramification 2-alkylique par distillation des produits de condensation des autres alcanals avec le formaldéhyde.

2. Procédé suivant la revendication 1, caractérisé en ce que l'on utilise le formaldéhyde en la proportion environ monomolaire ou en excès, par rapport à la somme des alcanals non substitués en poisition 2.

3. Procédé suivant la revendication 2, caractérisé en ce que l'on utilise de 0,5 à 1,5 équivalent d'acide carboxylique et de 0,05 à 1,5 mole d'amine, par rapport à une mole de formaldéhyde mis en oeuvre (calculé sur 100%) et on entreprend la réaction avec le formaldéhyde à un pH de 2,5 à 7.

4. Procédé suivant la revendication 1, caractérisé en ce que l'on récupère le 2-méthylbutanal du mélange des méthylbutanals isomériques et du valéraldéhyde.
